# EUROPÄISCHE PATENTSCHRIFT

(11) **EP 3 126 320 B1**
(45) Veröffentlichungstag und Bekanntmachung des Hinweises auf die Patenterteilung: **03.01.2018**
(21) Anmeldenummer: 15714783.6
(22) Anmeldetag: 27.03.2015
(51) Int. Cl.: C07C 67/05, C07C 67/48, C07C 69/15, B01D 53/14, B01D 53/62

(54) **VERFAHREN ZUR AUFREINIGUNG VON KOHLENDIOXID ENTHALTENDEN PROZESSGASEN AUS DER HERSTELLUNG VON VINYLACETAT**
PROCESS FOR THE PURIFICATION OF CARBON DIOXIDE CONTAINING PROCESS GASES FROM THE PREPARATION OF VINYL ACETATE
PROCÉDÉ POUR LA PURIFICATION DE GAZ DE PROCESSUS CONTENANT DU DIOXYDE DE CARBONE PROVENANT DE LA PRÉPARATION D' ACÉTATE DE VINYLE

(30) Priorität: 03.04.2014 DE 102014206450
(43) Veröffentlichungstag der Anmeldung: 08.02.2017
(73) Patentinhaber: Wacker Chemie AG, 81737 München (DE)
(72) Erfinder: BAUER, Markus, 84489 Burghausen (DE)
(74) Vertreter: Ege, Markus
(86) Internationale Anmeldenummer: PCT/EP2015/056804
(87) Internationale Veröffentlichungsnummer: WO 2015/150290

(56) Entgegenhaltungen:
- WO-A1-2010/124985
- DE-A1-102006 038 689
- US-A- 4 430 312
- WUNDER, R.: "CO2-Heißpottaschewäsche - Lehrbuch und Wirklichkeit", CHEMIE INGENIEUR TECHNIK, Bd. 60, Nr. 5, 1988, Seiten 410-412, XP55199514, ISSN: 0009-286X, DOI: 10.1002/cite.330600515
- AHMADI, M. ET AL.: "Advanced modelling in performance optimization for reactive separation in industrial CO2 removal", SEPARATION AND PURIFICATION TECHNOLOGY, Bd. 63, Nr. 1, 2008, Seiten 107-115, XP024528094, ISSN: 1383-5866, DOI: 10.1016/J.SEPPUR.2008.04.016 [gefunden am 2008-04-22]
- IMLE, M. ET AL.: "Solubility of Carbon Dioxide in Activated Potash Solutions in the Low and High Gas Loading Regions", INDUSTRIAL & ENGINEERING CHEMISTRY RESEARCH, Bd. 52, Nr. 37, 31. Juli 2013 (2013-07-31) , Seiten 13477-13489, XP55199570, ISSN: 0888-5885, DOI: 10.1021/ie401835x

## Beschreibung

Die Erfindung betrifft Verfahren zur Aufreinigung von Kohlendioxid enthaltenden Prozessgasen aus der Herstellung von Vinylacetat nach Umsetzung von Ethylen mit Essigsäure und Sauerstoff in heterogen katalysierten, kontinuierlichen Gasphasenprozessen.

Vinylacetat ist ein wichtiger Monomerbaustein für die Herstellung von Polymeren, wie beispielsweise Vinylacetat-Homopolymeren oder Vinylacetat-Mischpolymeren mit Ethylen, Vinylchlorid, Acrylaten, Maleinaten, Fumaraten oder Vinyllaurat. Die Herstellung von Vinylacetat erfolgt herkömmlicher Weise in einem Festbett-Röhrenreaktor (oder auch Wirbelschichtreaktor) in einer exothermen Umsetzung von Ethylen mit Essigsäure und Sauerstoff in heterogen katalysierten, kontinuierlichen Gasphasenprozessen. Hierbei werden üblicherweise Festbett-Katalysatoren eingesetzt, die im Allgemeinen Palladium- und Alkalimetallsalze auf einem Trägermaterial enthalten und zusätzlich noch mit Gold, Rhodium oder Cadmium dotiert sein können. Die Umsetzung wird üblicherweise bei einem Druck von 1 bis 30 bar und einer Temperatur von 130°C bis 200°C durchgeführt:

C₂H₄ + CH₃COOH + ½ O₂ -> CH₃COOCH=CH₂ + H₂O.

In einer Nebenreaktion wird Ethylen zu CO₂ (Kohlendioxid) oxidiert:

C₂H₄ + 3 O₂ -> 2 CO₂ + 2 H₂O.

Je größer der Anteil an Ethylen ist, der zu Nebenprodukten umgesetzt wird, desto geringer ist zwangsläufig die Selektivität der Umsetzung von Ethylen zu Vinylacetat. Erwünscht sind also möglichst hohe Ethylenselektivitäten.

Das dem Reaktor zugeführte Gasgemisch enthält im Allgemeinen einen mehrfach molaren Überschuss an Ethylen. Die Umsetzung von Ethylen und der weiteren Edukte im Reaktor verläuft unvollständig. Das dem Reaktor entnommene Prozessgas (Produktgasstrom) enthält im Wesentlichen Vinylacetat, Ethylen, Essigsäure, Wasser, Sauerstoff sowie Nebenprodukte, vorwiegend Kohlendioxid, und die Inerten Stickstoff, Argon, Methan und Ethan. Aus dem Produktgasstrom werden nach Verlassen des Reaktors das Reaktionsprodukt Vinylacetat, nicht umgesetzte Essigsäure, Wasser und weitere kondensierbare Anteile möglichst weitgehend auskondensiert und der weiteren Aufarbeitung und destillativen Reinigung zugeführt bis hin zur Isolierung des reinen Vinylacetats. Der nach Abtrennung von Vinylacetat und kondensierbaren Anteilen aus dem Produktgasstrom verbleibende Gasstrom wird als Kreisgas nach weiteren Reinigungsschritten schließlich wieder dem Reaktor zurückgeführt.

Besonderes Augenmerk bei der Reinigung des Kreisgases gilt der hinreichenden Ausschleusung des Nebenprodukts Kohlendioxid. Denn andernfalls kommt es während des kontinuierlichen Betriebs des Verfahrens zu einer Anreicherung von Kohlendioxid im Kreisgas, wodurch die Vinylacetatbildung im Reaktor binnen Kürze inhibiert werden würde. Deswegen wird in gängigen Verfahren ein Teil des Kreisgases vor der Rückführung in den Reaktor abgezweigt und zur Abtrennung von CO₂ der CO₂-Wäsche (CO₂-Absorption/Desorption) zugeführt und anschließend wieder mit dem weiteren Kreisgas vereint.

Das Kreisgas ist ein zu überwiegenden Anteilen aus Ethylen, Kohlendioxid, Ethan, Stickstoff und Sauerstoff bestehendes Gasgemisch. Das Kreisgas wird vor Rückführung in den Festbettröhrenreaktor mit den Reaktanden Essigsäure, Ethylen und Sauerstoff versetzt und mit Heizdampf betriebenen Wärmetauschern auf Reaktionstemperatur gebracht. Zur Kompensation von Druckverlusten wird das Kreisgas in der Regel vor Rückführung in den Reaktor mittels eines Kreisgasverdichters verdichtet.

Derartige Verfahren sind beispielsweise aus der DE-A1 102009002666 bekannt. Die DE-A 102006038689 beschreibt Verfahren zur Isolierung von Vinylacetat aus dem vorgenannten Produktgasstrom, wobei der im CO₂-Desorber ausgetriebene Wasserdampf energetisch verwertet wird. Auch die EP-A1 1760065 beschreibt Verfahren zur Isolierung von Vinylacetat aus dem Produktgasstrom, wobei die im Kreisgaswäscher anfallende essigsaure Lösung in einer bestimmten Weise im Verfahren zur Vinylacetat-Herstellung recycelt wird.

Auch vor dem Hintergrund dieser Entwicklungen besteht weiterhin Bedarf, die Abtrennung von Kohlendioxid aus dem Kreisgas effizienter, insbesondere weniger Energie-intensiv auszugestalten.

Die CO₂-Wäsche des Kreisgases wird seit vielen Jahrzehnten im Allgemeinen mit wässrigen Pottasche-Lösungen betrieben. Zur verbesserten Abtrennung von Kohlendioxid aus Gasen sind in den vergangenen Jahren auch im Zusammenhang mit der Speicherung von Kohlendioxid, auch als Carbon Capture and Storage (CCS) bezeichnet, eine Reihe von leistungsfähigeren Absorptionsmittel oder Additive entwickelt worden, wie beispielsweise Methylamin oder Ethanolamin. Solche Neuentwicklungen aus anderen Technologien sind aber nicht ohne weiteres auf Verfahren zur Aufreinigung des Kreisgases der Vinylacetat-Herstellung übertragbar, da hierbei in ein komplexes Gasgemisch eingegriffen wird, dessen Zusammensetzung seit Jahrzehnten immer weiter optimiert worden ist. Fatal wäre auch, wenn durch Modifizierung des Absorptionsmittels Verunreinigungen in das Kreisgas eingebracht und die weitere Verwertung oder Umsetzung des Kreisgases beeinträchtigen würden.

Vor diesem Hintergrund bestand die Aufgabe, die Abtrennung von CO₂ aus den Prozessgasen der Vinylacetat-Herstellung effizienter, insbesondere weniger Energie-intensiv zu gestalten.

Gegenstand der Erfindung sind Verfahren zur Aufreinigung von Kohlendioxid enthaltenden Prozessgasen aus der Herstellung von Vinylacetat nach Umsetzung von Ethylen mit Essigsäure und Sauerstoff in heterogen katalysierten, kontinuierlichen Gasphasenprozessen, dadurch gekennzeichnet, dass
Kohlendioxid enthaltende Prozessgase zur Entfernung von Kohlendioxid mit einer oder mehreren Waschlösungen in Kontakt gebracht werden, wobei ein oder mehrere Waschlösungen alkalische Medien sind und
I) ein oder mehrere Oxide von Metallen aus der Gruppe umfassend KVO₃, NaVO₃, KV₂O₅, Na₄V₂O₇ und K₄V₂O₇ sowie
II) ein oder mehrere Oxide von Metallen aus der Gruppe umfassend KBO₂, Na₂B₂O₄, K₂B₂O₄, Na₂B₂O₇ und K₂B₂O₇ enthalten.

Als Prozessgas kann jegliche Gaszusammensetzung des erfindungsgemäßen Verfahrens bezeichnet werden. Die konkrete Zusammensetzung und die weiteren Eigenschaften eines Prozessgases an einer bestimmten Stelle des Verfahrens werden durch die Verfahrensbedingungen festgelegt, nach denen das jeweilige Prozessgas generiert oder behandelt wurde. Entsprechende Angaben sind im Folgenden mit weiteren Details beschrieben.

Die Metalloxide oder deren Salze können in den Waschlösungen, in an sich bekannter Weise, als Monomere, Dimere oder Oligomere, dissoziert oder undissoziiert vorliegen.

Eine Waschlösung enthält vorzugsweise 0,5 bis 20 Gew.-%, besonders bevorzugt 2 bis 15 Gew.-% und am meisten bevorzugt 3 bis 10 Gew.-% Metalloxide, bezogen auf das Gesamtgewicht der Waschlösung. Die Metalloxide, insbesondere in den vorgenannten Mengen, sind in den Waschlösungen unter Normalbedingungen nach DIN50014 im Allgemeinen löslich.

Bevorzugte Waschlösungen enthalten 0,5 bis 15 Gew.-%, besonders bevorzugt 2 bis 10 Gew.-% und am meisten bevorzugt 3 bis 8 Gew.-% Metalloxide der Gruppe I und 1 bis 10 Gew.-%, besonders bevorzugt 1 bis 8 Gew.-% und am meisten bevorzugt 2 bis 6 Gew.-% Metalloxide der Gruppe II, je bezogen auf das Gesamtgewicht der Waschlösung. Das Gewichtsverhältnis der Metalloxide der Gruppe I zu den Metalloxiden der Gruppe II beträgt vorzugsweise 1:2 bis 4:1 und besonders bevorzugt 1:1 bis 2:1.

Die Waschlösungen sind im Allgemeinen wässrig. Die Waschlösungen enthalten vorzugsweise keine organischen Lösungsmittel, wie Amine, insbesondere keine Alkanol- oder Alkyl-Amine, wie Methylamine, Ethanolamine, Propanolamine oder Butanolamine.

Die Waschlösung ist ein alkalisches Medium, insbesondere eine alkalische Lösung. Die Waschlösungen enthalten vorzugsweise auch (Erd)Alkalihydroxide oder vorzugsweise (Erd)Alkalicarbonate, insbesondere Kaliumcarbonat. Die Waschlösungen enthalten vorzugsweise 15 bis 40 Gew.-% und besonders bevorzugt 20 bis 30 Gew.-% (Erd)Alkalihydroxide, bezogen auf das Gesamtgewicht einer Waschlösung.

Die Waschlösung befindet sich vorzugsweise in einem CO₂-Absorber. Der CO₂-Absorber wird im Folgenden auch als CO₂-Wäscher bezeichnet. CO₂-Wäsche steht für die erfindungsgemäße Aufreinigung von Prozessgasen mit CO₂-Wäschern, also für das Entfernen bzw. Abtrennen von Kohlendioxid aus einem Prozessgas. Als CO₂-Absorber können die auf dem vorliegenden technischen Gebiet gängigen Anlagen eingesetzt werden. CO₂-Absorber haben üblicherweise die Form von Kolonnen, insbesondere von Destillationskolonnen.

Das Prozessgas, aus dem Kohlendioxid entfernt werden soll, wird dem CO₂-Absorber gängigerweise im Bereich von dessen Sumpf zugeführt. Dem Kohlendioxid enthaltenden Prozessgas wird im Zuge des Passierens des CO₂-Absorbers Kohlendioxid entzogen. Kohlendioxid wird von der Waschlösung aufgenommen, beispielsweise chemisch oder physikalisch gebunden. Auf diese Weise kann das Prozessgas zumindest teilweise von Kohlendioxid befreit werden. Das so aufgereinigte Prozessgas wird üblicherweise am Kopf des CO₂-Absorbers entnommen und schließlich wieder dem Reaktor zugeführt zur weiteren Umsetzung zu Vinylacetat. Üblicherweise wird das Kreisgas vor Einbringen in den Reaktor mit Ethylen, Essigsäure und/oder Sauerstoff beaufschlagt sowie erforderlichenfalls verdichtet und/oder auf die gewünschte Temperatur gebracht.

Das Kohlendioxid enthaltende Prozessgas, das in den CO₂-Absorber eingebracht wird, enthält vorzugsweise 10 bis 20 Gew.-%, insbesondere 13 bis 19 Gew.-% Kohlendioxid, 55 bis 70 Gew.-%, insbesondere 60 bis 70 Gew.-% Ethylen sowie weitere, übliche Mengen an den Inerten, wie Ethan, Methan, Stickstoff oder Argon, sowie gegebenenfalls weitere Bestandteile, wobei sich die Angaben in Gew.-% auf das Gesamtgewicht dieses Prozessgases beziehen. Das aufgereinigte Prozessgas, das den CO₂-Absorber verlässt, enthält vorzugsweise 2,5 Gew.-%, besonders bevorzugt ≤ 2 Gew.-% und am meisten bevorzugt ≤ 1,6 Gew.-%, in der Regel jedoch ≥ 0,5 Gew.-% Kohlendioxid sowie vorzugsweise 70 bis 95 Gew.-% und besonders bevorzugt 75 bis 85 Gew.-% Ethylen und weitere, übliche Mengen an den Inerten, wie Ethan, Methan, Stickstoff oder Argon, sowie gegebenenfalls weitere übliche Bestandteile, wobei sich die Angaben in Gew.-% auf das Gesamtgewicht des aufgereinigten, den CO₂-Absorber verlassenden Prozessgases beziehen. Bei der erfindungsgemäßen Aufreinigung wird also insbesondere Kohlendioxid aus dem Prozessgas entfernt.

Der Druck des Prozessgases beim Eintreten in den CO₂-Absorber beträgt üblicherweise 9 bis 10 bar. Im CO₂-Absorber herrschen in der Regel Temperaturen von 95 bis 100°C.

Der Sumpf des CO₂-Absorbers, d.h. der Teil der Waschlösung, der am Sumpf des CO₂-Absorbers anfällt und mit Kohlendioxid beladen ist, wird üblicherweise einem CO₂-Desorber zugeführt. Die in den CO₂-Desorber eingeführte Waschlösung wird im CO₂-Desorber für gewöhnlich auf 100 bis 120°C erhitzt und von Kohlendioxid befreit. Freigesetztes Kohlendioxid wird aus dem Prozess ausgeschleust. Im Sumpf des CO₂-Absorbers fällt wie üblich eine zumindest teilweise von Kohlendioxid befreite Waschlösung an. Die am Sumpf des CO₂-Desorbers anfallende Waschlösung wird im Allgemeinen zumindest teilweise entnommen und im Bereich des Kopfes in den CO₂-Absorber zurückgeführt.

In einer bevorzugten Ausführungsform enthält der CO₂-Absorber oder der CO₂-Desorber Füllkörper, wie Pall-Ringe, Berl-Sattel, Hiflow-Ringe, Intalox-Sattel, Igel oder insbesondere Raschig-Ringe. Alternativ kann der CO₂-Absorber oder der CO₂-Desorber auch mit Stoffaustauschböden, bevorzugt Siebböden ausgestattet sein.

Ansonsten sind der CO₂-Absorbers und der CO₂-Desorber nach Stand der Technik aufgebaut und betrieben.

Der Wasserwäscher ist im Allgemeinen vor dem CO₂-Absorber in den Kreisprozess integriert, d.h. das Prozessgas wird üblicherweise zuerst durch einen Wasserwäscher geführt und anschließend in einen CO₂-Absorber eingebracht; oder anders ausgedrückt, der Wasserwäscher befindet sich im Allgemeinen zwischen der Stelle, an der dem Prozessgas ein Kreisgasteilstrom entnommen bzw. abgezweigt wird, und dem Eintritt dieses Kreisgasteilstromes in den CO₂-Absorber. Das Prozessgas wird üblicherweise im Bereich des Sumpfes in den Wasserwäscher eingeführt. Der Wasserwäscher wird im Allgemeinen mit Essigsäure und Wasser beschickt. Dadurch wird beispielsweise Vinylacetat aus dem Prozessgas gewaschen. Das so gereinigte Prozessgas kann am Kopf des Wasserwäschers entnommen und in den CO₂-Absorber eingebracht werden.

Im Folgenden ist das Verfahren zur Herstellung von Vinylacetat und die erfindungsgemäße Abtrennung von Kohlendioxid aus einem Prozessgas bzw. dem Kreisgas mit weiteren Details beschrieben.

Bei der kontinuierlichen Herstellung von Vinylacetat wird vorzugsweise in Röhrenreaktoren gearbeitet, welche mit einem Festbettkatalysator beschickt sind. Diese Katalysatoren sind im Allgemeinen mit Edelmetall(salz)en und Promotoren dotierte Trägerkatalysatoren, beispielsweise mit Palladium und mit Gold und Kalium-Salzen dotierte Bentonit-Kugeln. Der Reaktor wird mit Ethylen, Sauerstoff und Essigsäure beschickt und die Reaktion bei einem Druck von vorzugsweise 8 bis 12 bar abs. und einer Temperatur von vorzugsweise 130°C bis 200°C durchgeführt.

Die Reaktionstemperatur im Festbettröhrenreaktor von vorzugsweise 130°C bis 200°C wird mittels Siedewasserkühlung bei einem Druck von 1 bis 30 bar abs. eingestellt. Dabei wird Wasserdampf, der sogenannte Eigendampf, mit einer Temperatur von beispielsweise 120°C bis 185°C bei einem Druck von 1 bis 10 bar abs., vorzugsweise 2,5 bis 5 bar abs., gebildet. Das aus dem Reaktor austretende Produktgas enthält im Wesentlichen Vinylacetat, Ethylen, Essigsäure, Wasser, Sauerstoff, CO₂ sowie die Inerten Stickstoff, Argon, Methan und Ethan.

Im Allgemeinen wird das den Festbettröhrenreaktor verlassende Gasgemisch in eine Vorentwässerungskolonne geleitet und die am Sumpf der Kolonne anfallende flüssige Phase, hauptsächlich Vinylacetat, Essigsäure, Ethylacetat und Wasser, dem Rohvinylacetat-Sammelbehälter zugeführt. Üblicherweise wird in einer nachgeschalteten Azeotropkolonne in Vinylacetatmonomer (VAM) und Wasser als Kopfprodukt und Essigsäure als Sumpfprodukt aufgetrennt. Die Essigsäure kann in den Essigsäuresättiger geleitet und so in den Prozess zurückgeführt werden. Das als Kopfprodukt entnommene VAM kann über die Entwässerungskolonne zur Reinvinylacetatkolonne geleitet und dort in VAM und Essigsäure getrennt werden.

Das am Kopf der Vorentwässerungskolonne entnommene gasförmige Gemisch, bestehend im Wesentlichen aus Ethylen und CO₂, wird üblicherweise im Kreisgaswäscher von weiteren, nach Möglichkeit von allen kondensierbaren Anteilen befreit. Das am Kopf des Kreisgaswäschers entnommene Kreisgas wird üblicherweise im Kreisgasverdichter, zum Ausgleich der im Reaktionskreislauf auftretenden Druckverluste, verdichtet und wieder als Kreisgas in den Reaktor zurückgeführt. Das Druckniveau des Kreisgases beträgt vorzugsweise 8 bis 12 bar abs.

Ein Teilstrom des Kreisgases (Kreisgasteilstrom) wird vorzugsweise auf der Saugseite oder Druckseite, insbesondere der Druckseite, des Kreisgasverdichters abgezweigt und zur erfindungswesentlichen CO₂-Entfernung der CO₂-Wäsche zugeführt und geht nach der CO₂-Entfernung wieder wie üblich druckseitig zurück zum Verdichter. Der am Kreisgasverdichter entnommene Teilstrom macht beispielsweise 8 bis 20 Vol.-% des gesamten Kreisgases aus. In alternativen Ausgestaltungen des Verfahrens können am Kreisgasverdichter auch 8 bis 12 Vol.-% oder mehr als 12 bis 18 Vol.-% des gesamten Kreisgases entnommen werden. Üblicherweise wird nach der Abzweigung und vor der CO₂-Wäsche der entnommene Teilstrom in einer Kolonne (Wasserwäscher) unter Zuführung von Wasser und Essigsäure gewaschen. Das flüssige Sumpfprodukt kann im Rohvinylacetatbehälter gesammelt werden und in der nachgeschalteten Azeotropkolonne aufgetrennt werden. Das gasförmige Kopfprodukt des Wasserwäschers, im Wesentlichen Ethylen und CO₂, wird der CO₂-Wäsche zugeführt.

Der Kreisgasteilstrom (Kopfprodukt des Wasserwäschers) wird nun vorzugsweise in einen CO₂-Absorber, auch CO₂-Wäscher genannt, geführt.

Der Kreisgasteilstrom wird im Allgemeinen nach der CO₂-Wäsche, auf der Druckseite des Kreisgasverdichters, und stromabwärts von der Entnahmestelle des Teilstroms zur CO₂-Wäsche, mit dem weiteren Kreisgas vereint. Im Allgemeinen wird zum Ausgleich des Druckverlustes der Kreisgasteilstrom mittels Ethylen, welches beispielsweise mit einem Druck von 15 bis 25 bar abs. vorliegt, auf ein Druckniveau von vorzugsweise 0,5 bis 2 bar über dem Druck des Kreisgases gebracht, und dem Kreisgas zugeführt. Vorzugsweise wird der Kreisgasteilstrom mit der erforderlichen Menge Ethylen über einen Strahlverdichter (Ejektoren, Injektoren), vorzugsweise eine Saugdüse, zugeführt. In einer bevorzugten Ausführungsform kann auch so vorgegangen werden, dass der gesamte Ethylenfeed, welcher vor dem Reaktor dem Kreisgas zugeführt wird, über den Strahlverdichter zugeführt wird.

Mit dem erfindungsgemäßen Einsatz von Metalloxiden wird eine erhebliche Produktionssteigerung und Energieeinsparung bei der Gewinnung und Reinigung von Vinylacetat erzielt. So kann bei der erfindungsgemäßen Vorgehensweise im Vergleich zu herkömmlichen Verfahren der Anteil des Kreisgases, der im CO₂-Wäscher zu reinigen ist, reduziert werden, ohne Einbußen am Reinheitsgrad oder der Zusammensetzung des Kreisgases hinnehmen zu müssen. Da auf diese Weise weniger Flüssigkeitsmengen umgewälzt werden müssen, ist hiermit eine erhebliche Einsparung an Equipment und Energie verbunden. So ist beispielsweise der Energieaufwand für das Umpumpen der Flüssigkeit und der Bedarf an Heizdampf reduziert.

Wenn im erfindungsgemäßen Verfahren alternativ dieselben Anteile des Kreisgases in einem CO₂-Wäscher aufgereinigt werden wie im Stand der Technik, d.h. wenn derselbe Aufwand betrieben wird wie im Stand der Technik, so wird bei der erfindungsgemäßen Vorgehensweise im Vergleich zur herkömmlichen Vorgehenswiese ein größerer Reinigungseffekt erzielt. Dadurch kann Kreisgas mit höherem Ethylengehalt gewonnen werden und Vinylacetat effizienter hergestellt werden. Diese Vorteile kommen auch zum Tragen, wenn die Kapazität einer Anlage zur Vinylacetatherstellung gesteigert werden soll und somit größere Mengen am Nebenprodukt Kohlendioxid anfallen und aus dem Kreisprozess auszuschleusen sind.

Vorteilhafterweise werden in Folge des erfindungsgemäßen Einsatzes von Metalloxiden keine Stoffe oder zumindest keine nennenswerten Mengen an Stoffen, wie Katalysatorgifte, in das Kreisgas eingebracht, die für dessen weitere Umsetzung im Reaktor zu Vinylacetat schädlich sind.

Die nachfolgenden Beispiele dienen der weiteren Illustration der Erfindung.

### Allgemeine Verfahrensbeschreibung:

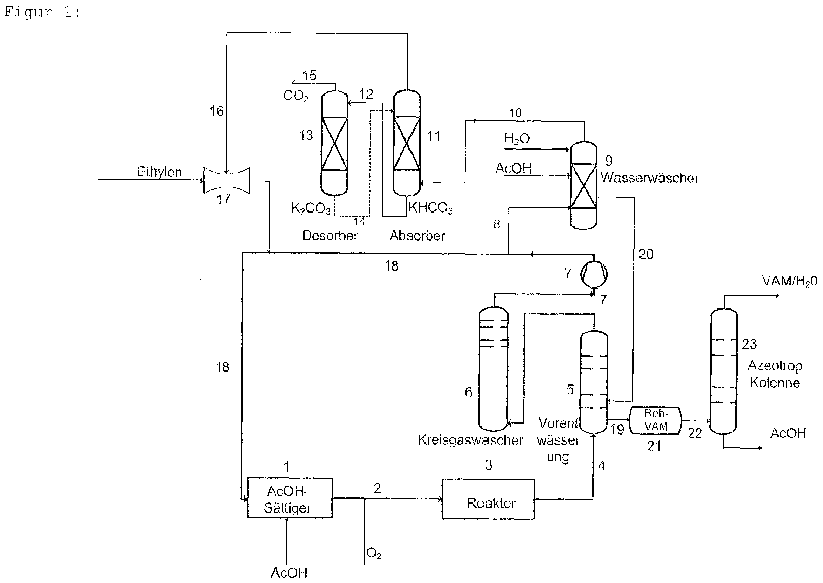

In einer Anlage gemäß Figur 1 wurde ethylenhaltiges Kreisgas im Essigsäuresättiger 1 (AcOH-Sättiger) mit Essigsäure (AcOH) beschickt, danach Sauerstoff (O₂) hinzugefügt und über eine mit Dampf beheizte Leitung 2 dem Röhrenreaktor 3 zugeführt. Das den Reaktor verlassende Kreisgas-Gemisch, welches im Wesentlichen Ethylen, Vinylacetat, Essigsäure, Kohlendioxid, Sauerstoff sowie Inerte enthielt, wurde über Leitung 4 der Vorentwässerungskolonne 5 zugeführt. In der Vorentwässerungskolonne 5 wurde das Gemisch aufgetrennt, wobei das Sumpfprodukt enthaltend im Wesentlichen VAM, Essigsäure und Wasser (H₂O) über Leitung 19 dem Rohvinylacetat-Behälter 21 zugeführt wurde, und nach Transfer über Leitung 22 in die Azeotropkolonne 23 in eine VAM-Fraktion und eine Essigsäure-Fraktion aufgetrennt wurde, welche jeweils, in hier nicht dargestellten Prozessschritten weiter aufgearbeitet wurden.
Das Kopfprodukt der Vorentwässerungskolonne 5 wurde entnommen und im nachgeschalteten Kreisgaswäscher 6 mittels Waschen mit Essigsäure von gasförmigem VAM befreit. Das Gasgemisch (Kreisgas) wurde mit dem Kreisgas-Verdichter 7 um etwa 3 bar höherverdichtet. Der Großteil des Kreisgases wurde über Leitung 18 in den Essigsäuresättiger 1 zurückgeführt.
Ein Anteil von 12 bzw. 18 Vol.-% des Kreisgases, wie in der Tabelle für das jeweilige (Vergleichs)Beispiel angegeben, wurde druckseitig vom Kreisgasverdichter 7 abgezweigt und über Leitung 8 in den Wasserwäscher 9 überführt und dort, zur Entfernung von weiterem Vinylacetat, mit Essigsäure und anschließend Wasser behandelt. Das Essigsäure, Wasser und Vinylacetat umfassende Sumpfprodukt wurde über Leitung 20 direkt in die Vorentwässerungskolonne 5 eingeleitet.
Das Kopfprodukt des Wasserwäschers 9 wurde über Leitung 10 im Bereich des Sumpfes mit einer Rate von 10 Tonnen pro Stunde in die CO₂-Absorptionskolonne 11 geleitet. Die CO₂-Absorptions-kolonne 11 wurde mit einer Umlaufrate 90 Tonnen pro Stunde bzw. 45 Tonnen pro Stunde einer 25 Gew.-%igen wässrigen Kaliumcarbonat-Waschlösung betrieben, die gegebenenfalls 6 Gew.-% Kaliumvanadat und 4 Ges.-% Kaliumborat als Zusätze enthielt - wie in der Tabelle näher spezifiziert.
Ansonsten wurden die CO₂-Absorptionskolonne 11 und die CO₂-Desorptionskolonne 13 in herkömmlicher Weise betrieben.
Der Sumpf der CO₂-Absorptionskolonne 11 enthielt wie üblich Kaliumhydrogencarbonat und wurde über Leitung 12 in den Kopf der CO₂-Desorptionskolonne 13 geleitet. Der Sumpf der CO₂-Desorptionskolonne 13 enthielt Kaliumcarbonat und wurde über Leitung 14 im Bereich des Kopfes in die CO₂-Absorptionskolonne 11 eingeführt. Die von der CO₂-Absorptionskolonne 11 in die Desorptionskolonne 13 überführten Massestrommengen entsprachen den von der Desorptionskolonne 13 in die CO₂-Absorptionskolonne 11 überführten Massestrommengen. Die in die CO₂-Desorptionskolonne 13 eingebrachte Waschlösung wurde mittels Heizdampf erhitzt und dadurch von Kohlendioxid befreit. Die im jeweiligen (Vergleichs)Beispiel eingesetzten Heizdampfmengen sind in der Tabelle angegeben. Über Leitung 15 wurde CO₂ aus der Desorptionskolonne 13 entfernt.
Das Kopfprodukt der CO₂-Absorptionskolonne 11 wurde über die Leitung 16 in den Strahlsauger 17 geleitet, dort mit Ethylenfeed versetzt und schließlich über den Essigsäuresättiger 1 in den Reaktor 3 eingeleitet.

Aus Vergleichsbeispiel 1 (VBsp.1) und Beispiel 2 (Bsp.2) geht hervor, dass der erfindungsgemäße Zusatz von Kaliumvanadat und Kaliumborat die Effizienz der Kreisgaswäsche in der CO₂-Absorptionskolonne 11 erheblich steigert. Dadurch konnten die Umlaufrate der Kaliumcarbonat-Lösung Waschlösung in der CO₂-Absorptionskolonne 11 und die erforderliche Heizdampfmenge in der CO₂-Desorptionskolonne 13 halbiert werden, und dies bei gleichbleibender Abrennung von Kohlendioxid, wie die CO₂-Konzentration im Kreisgas vor Eintritt in den Essigsäuresättiger 1 in der Tabelle anzeigt. Dadurch kann der Energie-Bedarf beim erfindungsgemäßen Verfahren im Vergleich zu herkömmlichen Verfahren reduziert werden.

**Tabelle:**

| | VBsp.1 | Bsp.2 | Bsp.3 |
|---|---|---|---|
| Anteil des in den Wasserwäscher 9 überführten Kreisgases [Vol.-%] | 12 | 12 | 18 |
| Umlaufrate der Kaliumcarbonat-Waschlösung in der CO₂-Absorptionskolonne 11 [t/h] | 90 | 45 | 90 |
| Zusätze der Waschlösung: | | | |
| Kalium-Vanadat KVO₃ | - | + | + |
| Kalium-Borat KBO₂ | - | + | + |
| in die CO₂-Desorptionskolonne 13 eingebrachte Heizdampfmenge [t/h] | 4,5 | 2,3 | 4,5 |
| CO₂-Konzentration des Prozessgases, das dem Wasserwäscher 9 über Leitung 8 zugeführt wurde [Vol.-%] | 20 | 20 | 20 |
| CO₂-Konzentration im Kreisgas vor Eintritt in den Essigsäuresättiger 1 [Vol.-%] | 18,3 | 18,3 | 17,5 |

Der Vergleich von Beispiel 3 (Bsp.3) und Vergleichsbeispiel 1 zeigt, dass die Kapazität der erfindungsgemäßen Waschlösung im Vergleich zu einer herkömmlichen Waschlösung erheblich gesteigert ist, so dass bei ansonsten gleicher Betriebsweise wie im Stand der Technik 50% mehr an Kreisgas über den Wasserwäscher 9 in die CO₂-Absorptionskolonne 11 eingebracht und von Kohlendioxid befreit werden kann, ohne dass dadurch zusätzlicher apparativer oder energetischer Aufwand entsteht. Dadurch kann letztlich der Ethylengehalt des Kreisgases beispielsweise vor Eintritt in den Essigsäuresättiger 1 und damit auch bei Eintritt in den Reaktor gesteigert werden. Konkret bedeutet dies, dass im Beispiel 3 durch Einbringen eines größeren Anteils des Kreisgases in den Wasserwäscher 9 und damit auch in die CO₂-Absorptionskolonne 11 in der dortigen Reinigung der Kohlendioxidanteil von 19 Gew.-% auf unter 3 Gew.-% Kohlendioxid gesenkt wurde und insgesamt 50% mehr Kohlendioxid entfernt wurden als im Vergleichsbeispiel 1. Dies hatte letztlich zur Folge, dass der Kohlendioxid-Gehalt im Kreisgas von 18,3 Gew.-% im Vergleichsbeispiel 1 auf 17,5 Gew.-% in Beispiel 3 abgereinigt wurde. Das nicht mehr von Kohlendioxid beanspruchte Volumen des Kreisgases wurde am Strahlsauger 17 von Ethylen aufgefüllt, so dass der Ethylengehalt vor Eintritt in den Essigsäuresättiger 1 um 0,8 Gew.-% gesteigert wurde.

## Patentansprüche

1. Verfahren zur Aufreinigung von Kohlendioxid enthaltenden Prozessgasen aus der Herstellung von Vinylacetat nach Umsetzung von Ethylen mit Essigsäure und Sauerstoff in heterogen katalysierten, kontinuierlichen Gasphasenprozessen, **dadurch gekennzeichnet, dass**
Kohlendioxid enthaltende Prozessgase zur Entfernung von Kohlendioxid mit einer oder mehreren Waschlösungen in Kontakt gebracht werden, wobei
ein oder mehrere Waschlösungen alkalische Medien sind und
I) ein oder mehrere Oxide von Metallen aus der Gruppe umfassend KVO₃, NaVO₃, KV₂O₅, Na₄V₂O₇ und K₄V₂O₇ sowie
II) ein oder mehrere Oxide von Metallen aus der Gruppe umfassend KBO₂, Na₂B₂O₄, K₂B₂O₄, Na₂B₂O₇ und K₂B₂O₇
enthalten.

2. Verfahren zur Aufreinigung von Kohlendioxid enthaltenden Prozessgasen aus der Herstellung von Vinylacetat nach Anspruch 1, **dadurch gekennzeichnet, dass**
sich eine Waschlösung gemäß Anspruch 1 in einem CO₂-Absorber befindet und
ein Prozessgas, aus dem Kohlendioxid entfernt werden soll, dem CO₂-Absorber im Bereich von dessen Sumpf zugeführt wird und dem Kohlendioxid enthaltenden Prozessgas im Zuge des Passierens des CO₂-Absorbers Kohlendioxid entzogen und das Kohlendioxid von der Waschlösung aufgenommen wird und das so aufgereinigte Prozessgas am Kopf des CO₂-Absorbers entnommen und schließlich wieder dem Reaktor zugeführt wird zur weiteren Umsetzung zu Vinylacetat.

3. Verfahren zur Aufreinigung von Kohlendioxid enthaltenden Prozessgasen aus der Herstellung von Vinylacetat nach Anspruch 1 bis 2, **dadurch gekennzeichnet, dass** ein oder mehrere Waschlösungen 0,5 bis 20 Gew.-% Oxide von Metallen enthalten, bezogen auf das Gesamtgewicht der Waschlösung.

4. Verfahren zur Aufreinigung von Kohlendioxid enthaltenden Prozessgasen aus der Herstellung von Vinylacetat nach Anspruch 1 bis 3, **dadurch gekennzeichnet, dass** ein oder mehrere Waschlösungen
0,5 bis 15 Gew.-% Oxide von Metallen der Gruppe I und
1 bis 10 Gew.-% Oxide von Metallen der Gruppe II enthalten, je bezogen auf das Gesamtgewicht der Waschlösung.

5. Verfahren zur Aufreinigung von Kohlendioxid enthaltenden Prozessgasen aus der Herstellung von Vinylacetat nach Anspruch 1 bis 4, **dadurch gekennzeichnet, dass** das Gewichtsverhältnis der Oxide von Metallen der Gruppe I zu den Oxiden von Metallen der Gruppe II 1:2 bis 4:1 beträgt.

6. Verfahren zur Aufreinigung von Kohlendioxid enthaltenden Prozessgasen aus der Herstellung von Vinylacetat nach Anspruch 1 bis 5, **dadurch gekennzeichnet, dass** ein oder mehrere Waschlösungen Erdalkalihydroxide, Alkalihydroxide, Erdalkalicarbonate oder Alkalicarbonate enthalten.

7. Verfahren zur Aufreinigung von Kohlendioxid enthaltenden Prozessgasen aus der Herstellung von Vinylacetat nach Anspruch 1 bis 6, **dadurch gekennzeichnet, dass** sich eine Waschlösung in einem CO₂-Absorber befindet und das aufgereinigte Prozessgas, das den CO₂-Absorber verlässt, ≤ 1,6 Gew.-% Kohlendioxid, 70 bis 95 Gew.-% Ethylen und weitere, übliche Mengen an Inerten ausgewählt aus der Gruppe umfassend Ethan, Methan, Stickstoff und Argon sowie gegebenenfalls weitere übliche Bestandteile enthält, wobei sich die Angaben in Gew.-% auf das Gesamtgewicht des aufgereinigten, den CO₂-Absorber verlassenden Prozessgases beziehen.

## Claims

1. Process for cleaning carbon dioxide-containing process gases from the preparation of vinyl acetate after reaction of ethylene with acetic acid and oxygen in heterogeneously catalyzed continuous gas-phase processes, **characterized in that**
carbon dioxide-containing process gases, for removing carbon dioxide, are contacted with one or more scrubbing solutions, wherein
one or more scrubbing solutions are alkaline media and contain
I) one or more oxides of metals form the group comprising KVO₃, NaVO₃, KV₂O₅, Na₄V₂O₇ and K₄V₂O₇ and also
II) one or more oxides of metals of the group comprising KBO₂, Na₂B₂O₄, K₂B₂O₄, Na₂B₂O₇ and K₂B₂O₇.

2. Process for cleaning carbon dioxide-containing process gases from the preparation of vinyl acetate according to Claim 1, **characterized in that**
a scrubbing solution according to Claim 1 is situated in a CO₂ absorber and
a process gas from which carbon dioxide is to be removed is fed to the CO₂ absorber in the region of the sump thereof and carbon dioxide is taken off from the carbon dioxide-containing process gas in the course of passage through the CO₂ absorber and the carbon dioxide is taken up by the scrubbing solution and the process gas thus cleaned is withdrawn at the top of the CO₂ absorber and is finally fed back to the reactor for further reaction to form vinyl acetate.

3. Process for cleaning carbon dioxide-containing process gases from the preparation of vinyl acetate according to Claim 1 to 2, **characterized in that** one or more scrubbing solutions contain 0.5 to 20% by weight of oxides of metals, based on the total weight of the scrubbing solution.

4. Process for cleaning carbon dioxide-containing process gases from the preparation of vinyl acetate according to Claim 1 to 3, **characterized in that** one or more scrubbing solutions contain
0.5 to 15% by weight of oxides of metals of group I and
1 to 10% by weight of oxides of metals of group II, in each case based on the total weight of the scrubbing solution.

5. Process for cleaning carbon dioxide-containing process gases from the preparation of vinyl acetate according to Claim 1 to 4, **characterized in that** the weight ratio of the oxides of metals of group I to the oxides of metals of group II is 1:2 to 4:1.

6. Process for cleaning carbon dioxide-containing process gases from the preparation of vinyl acetate according to Claim 1 to 5, **characterized in that** one or more scrubbing solutions contain alkaline earth metal hydroxides, alkali metal hydroxides, alkaline earth metal carbonates or alkali metal carbonates.

7. Process for cleaning carbon dioxide-containing process gases from the preparation of vinyl acetate according to Claim 1 to 6, **characterized in that** a scrubbing solution is situated in a CO₂ absorber and the cleaned process gas that leaves the CO₂ absorber contains ≤ 1.6% by weight of carbon dioxide, 70 to 95% by weight of ethylene and further usual amounts of inerts selected from the group comprising ethane, methane, nitrogen and argon, and also optionally further usual components, wherein the figures in % by weight refer to the total weight of the cleaned process gas leaving the CO₂ absorber.

## Revendications

1. Procédé pour la purification de gaz de procédé, contenant du dioxyde de carbone, provenant de la préparation d'acétate de vinyle après la transformation d'éthylène avec de l'acide acétique et de l'oxygène dans des procédés en phase gazeuse, continus, catalysés par voie hétérogène, **caractérisé en ce que** les gaz de procédé contenant du dioxyde de carbone sont mis en contact avec une ou plusieurs solutions de lavage en vue de l'élimination du dioxyde de carbone, une ou plusieurs solutions de lavage étant des milieux alcalins et contenant
I) un ou plusieurs oxydes de métaux du groupe comprenant KVO₃, NaVO₃, KV₂O₅, Na₄V₂O₇ et K₄V₂O₇ ainsi que
II) un ou plusieurs oxydes de métaux du groupe comprenant KBO₂, Na₂B₂O₄, K₂B₂O₄, Na₂B₂O₇ et K₂B₂O₇.

2. Procédé pour la purification de gaz de procédé, contenant du dioxyde de carbone, provenant de la préparation d'acétate de vinyle selon la revendication 1, **caractérisé en ce qu'**une solution de lavage selon la revendication 1 se trouve dans un absorbeur de CO₂ et un gaz de procédé, dont le dioxyde de carbone doit être éliminé, est introduit dans l'absorbeur de CO₂ au niveau de son fond et du dioxyde de carbone est soutiré du gaz de procédé contenant du dioxyde de carbone au cours de son passage dans l'absorbeur de CO₂ et le dioxyde de carbone est absorbé par la solution de lavage et le gaz de procédé ainsi purifié est prélevé en tête de l'absorbeur du CO₂ et enfin de nouveau introduit dans le réacteur pour la transformation ultérieure en acétate de vinyle.

3. Procédé pour la purification de gaz de procédé, contenant du dioxyde de carbone, provenant de la préparation d'acétate de vinyle selon la revendication 1 à 2, **caractérisé en ce qu'**une ou plusieurs solutions de lavage contiennent 0,5 à 20% en poids d'oxyde de métaux, par rapport au poids total de la solution de lavage.

4. Procédé pour la purification de gaz de procédé, contenant du dioxyde de carbone, provenant de la préparation d'acétate de vinyle selon la revendication 1 à 3, **caractérisé en ce qu'**une ou plusieurs solutions de lavage contiennent 0,5 à 15% en poids d'oxyde de métaux du groupe I et 1 à 10% en poids d'oxyde de métaux du groupe II, à chaque fois par rapport au poids total de la solution de lavage.

5. Procédé pour la purification de gaz de procédé, contenant du dioxyde de carbone, provenant de la préparation d'acétate de vinyle selon la revendication 1 à 4, **caractérisé en ce que** le rapport pondéral des oxydes de métaux du groupe I aux oxydes de métaux du groupe II est de 1:2 à 4:1.

6. Procédé pour la purification de gaz de procédé, contenant du dioxyde de carbone, provenant de la préparation d'acétate de vinyle selon la revendication 1 à 5, **caractérisé en ce qu'**une ou plusieurs solutions de lavage contiennent des hydroxydes de métal alcalino-terreux, des hydroxydes de métal alcalin, des carbonates de métal alcalino-terreux ou des carbonates de métal alcalin.

7. Procédé pour la purification de gaz de procédé, contenant du dioxyde de carbone, provenant de la préparation d'acétate de vinyle selon la revendication 1 à 6, **caractérisé en ce qu'**une solution de lavage se trouve dans un absorbeur de CO₂ et le gaz de procédé purifié, qui quitte l'absorbeur de CO₂, contient ≤ 1,6% en poids de dioxyde de carbone, 70 à 95% en poids d'éthylène et d'autres quantités usuelles d'inertes, choisis dans le groupe comprenant l'éthane, le méthane, l'azote et l'argon ainsi que le cas échéant d'autres constituants usuels, les indications en % en poids se rapportant au poids total du gaz de procédé purifié, quittant l'absorbeur de CO₂.
